(19) Europäisches Patentamt
European Patent Office
Office européen des brevets

(11) Veröffentlichungsnummer: **0 014 959**
**A1**

(12) # EUROPÄISCHE PATENTANMELDUNG

(21) Anmeldenummer: **80100738.6**

(22) Anmeldetag: **14.02.80**

(51) Int. Cl.³: **C 07 D 487/04**
A 61 K 31/55
//C07D231/38, C07D231/40,
(C07D487/04, 243/00, 231/00)

(30) Priorität: **20.02.79 DE 2906401**

(43) Veröffentlichungstag der Anmeldung:
**03.09.80 Patentblatt 80/18**

(84) Benannte Vertragsstaaten:
**AT BE CH DE FR GB IT NL SE**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Rackur, Gerhard, Dr.**
**Gundelhardtstrasse 2**
**D-6233 Kelkheim (Taunus)(DE)**

(72) Erfinder: **Hoffmann, Irmgard, Dr.**
**Oranienstrasse 1**
**D-6232 Bad Soden am Taunus(DE)**

(54) **8-Aryl-5,6,7,8-tetrahydropyrazolo(3,4-b) (1,5)diazepin-1H,4H-5,7-dione und Verfahren zu ihrer Herstellung und diese enthaltende Arzneimittel.**

(57) 8-Aryl-5,6,7,8-tetrahydropyrazolo (3,4-b) (1,5)diazepin-1H,4H-5,7-dione der Formel

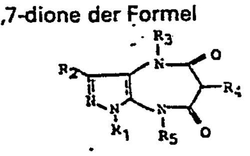

worin
$R_1$ und $R_2$ Wasserstoffatome, Alkylgruppen darstellen, wobei einer der Reste $R_1$ und $R_2$ jeweils auch eine Benzyl- oder Phenylgruppe sein kann, $R_3$ ein Wasserstoffatom, eine Alkylgruppe, die gegebenenfalls durch eine Alkoxygruppe oder eine Dialkylaminogruppe oder eine Cycloalkylgruppe substituiert ist, oder eine Alkenyl- oder Alkinylgruppe, eine Cycloalkylgruppe, oder eine Carbalkoxygruppe bedeutet, $R_4$ ein Wasserstoffatom, eine Alkylgruppe, eine Phenylgruppe, eine Hydroxygruppe, eine Alkoxygruppe, eine Carbalkoxygruppe, eine Acylgruppe, eine Aminogruppe, eine Alkylaminogruppe, oder eine Dialkylaminogruppe, oder eine Carbamoylgruppe, die eine Aminogruppe, eine Alkylaminogruppe, oder eine Dialkylaminogruppe trägt, ist. $R_5$ eine Phenylgruppe, eine ein- oder zweifach durch Methyl, Cl, Br, F, Nitro, Cyan und/oder Trifluormethyl substituierte Phenylgruppe oder eine 2-Pyridylgruppe sein kann;
Verfahren zu ihrer Herstellung und diese enthaltende Arzneimittel mit anxiolytischer Wirksamkeit.

EP 0 014 959 A1

Croydon Printing Company Ltd.

BAD ORIGINAL

8-Aryl-5,6,7,8-tetrahydropyrazolo(3,4-b) (1,5)diazepin-1H,4H-5,7-dione und Verfahren zu ihrer Herstellung und diese enthaltende Arzneimittel

Die Erfindung betrifft 8-Aryl-5,6,7,8-tetrahydropyrazolo (3,4-b)(1,5)diazepin-1H,4H-5,7-dione der Formel

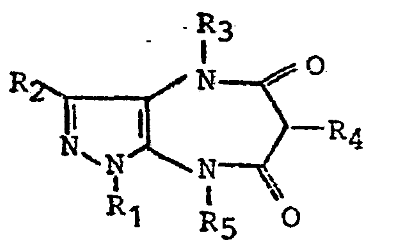

I

worin

$R_1$ und $R_2$ gleich oder verschieden sind und Wasserstoffatome, Alkylgruppen mit 1-6 C-Atomen darstellen, wobei einer der Reste $R_1$ und $R_2$ jeweils auch eine Benzyl- oder Phenylgruppe sein kann,

$R_3$ ein Wasserstoffatom, eine Alkylgruppe mit 1-6 C-Atomen, die gegebenenfalls durch eine Alkoxygruppe mit 1-6 C-Atomen oder eine Dialkylaminogruppe mit 2-12 C-Atomen oder eine Cycloalkylgruppe mit 3-6 C-Atomen substituiert ist, oder eine Alkenyl- oder Alkinylgruppe mit 2-6 C-Atomen, eine Cycloalkylgruppe mit 3-6 C-Atomen, oder eine Carbalkoxygruppe mit 2-6 C-Atomen bedeutet,

$R_4$ ein Wasserstoffatom, eine Alkylgruppe mit 1-6 C-Atomen,

eine Phenylgruppe, eine Hydroxygruppe, eine Alkoxygruppe mit 1-6 C-Atomen, eine Carbalkoxygruppe mit 2-6 C-Atomen, eine Acylgruppe mit 2-6 C-Atomen, eine Aminogruppe, eine Alkylaminogruppe mit 1-7 C-Atomen oder eine Dialkylaminogruppe mit 2-12 C-Atomen oder eine Carbamoylgruppe, die eine Aminogruppe, eine Alkylaminogruppe mit 1-6 C-Atomen oder eine Dialkylaminogruppe mit 2-12 C-Atomen trägt, ist,

$R_5$ eine Phenylgruppe, eine ein- oder zweifach durch Methyl, Cl, Br, F, Nitro, Cyan und/oder Trifluormethyl substituierte Phenylgruppe oder eine 2-Pyridylgruppe sein kann.

Insbesondere beinhaltet die Erfindung Verbindungen, worin $R_1$ und $R_2$ gleich oder verschieden sind und Wasserstoff, Methyl-, Ethyl-, iso-Propyl-, n-Butyl bedeuten und $R_1$ ein Phenyl- oder Benzylrest sein kann.

Für den Rest $R_3$ kommen insbesondere ein Wasserstoffatom, eine Methyl-, Ethyl-, Propenyl-, Propinyl-, Cyclopropylmethylgruppe, eine Methoxymethylen- oder eine Ethoxymethylengruppierung in Betracht.

Bei dem Rest $R_4$ handelt es sich bevorzugt um Wasserstoff, eine Methyl-, Ethyl-, iso-Propyl- oder n-Butylgruppe oder eine Hydroxy- oder Alkoxygruppe mit 1-4 C-Atomen oder eine Carbalkoxygruppe mit 2-4 C-Atomen.

Für $R_5$ kommen insbesondere o-Chlorphenyl, m-Chlorphenyl, p-Chlorphenyl-, 2,4-Dichlorphenylgruppen sowie die entsprechenden Fluorderivate in Betracht.

Besonders günstige Eigenschaften besitzen solche Verbindungen, der Formel I bei denen $R_1$ Methyl, Ethyl oder Phenyl bedeutet, $R_2$ ein Methylrest ist, $R_3$ Wasserstoff, Methyl, Ethyl, Cyclopropylmethyl oder Propinyl ist, $R_4$ Wasserstoff bedeutet und $R_5$ ein Phenyl- oder ein o- oder m-Chlorphenylrest ist.

Verbindungen gemäß der Erfindung sind beispielsweise:

1-Methyl-8-phenyl-5,6,7,8-tetrahydropyrazolo(3,4-b)(1,5) diazepin-1H,4H-5,7-dion, 1,4-Dimethyl-8-phenyl-5,6,7,8-tetrahydropyrazolo(3,4-b)(1,5)diazepin-1H,4H-5,7-dion, 4-Ethyl-1-methyl-8-phenyl-5,6,7,8-tetrahydropyrazolo(3,4-b)(1,5)diazepin-1H,4H-5,7-dion, 4-Allyl-1methyl-8-phenyl-5,6,7,8-tetrahydropyrazolo(3,4-b)(1,5) diazepin-1H,4H-5,7-dion, 1-Methyl-4-(2-propinyl)-8-phenyl-5,6,7,8-tetrahydropyrazolo-(3,4-b)(1,5)diazepin-1H,4H-5,7-dion, 4-Cyclopropylmethyl-1-methyl-8-phenyl-5,6,7,8-tetrahydropyrazolo(3,4-b)(1,5) diazepin-1H,4H-5,7-dion, 1-Methyl-4-(2-dimethylaminoethyl)-8-phenyl-5,6,7,8-tetrahydropyrazolo-(3,4-b)(1,5)diazepin-1H,4H-5,7-dion, 4-(2-Diethylaminoethyl)-1-methyl-8-phenyl-5,6,7,8-tetrahydropyrazolo(3,4-b)(1,5)diazepin-1H,4H-5,7-dion, 4-(2,2,2-trifluorethyl)-1-methyl-8-phenyl-5,6,7,8-tetrahydropyrazolo(3,4-b)(1,5)diazepin-1H,4H-5,7-dion, 1-Methyl-4-(2-methylsulfonylethyl)-8-phenyl-5,6,7,8-tetrahydropyrazolo(3,4-b)(1,5)diazepin-1H,4H-5,7-dion, 1,3-Dimethyl-4-ethyl-8-phenyl-5,6,7,8-tetrahydropyrazolo(3,4-b)(1,5)diazepin-1H,4H-5,7-dion, 4-Allyl-1,3-dimethyl-8-phenyl-5,6,7,8-tetrahydropyrazolo(3,4-b)(1,5)diazepin-1H,4H-5,7-dion, 1,3-Dimethyl-4-(2-propinyl)-8-phenyl-5,6,7,8-tetrahydropyrazolo(3,4-b)(1,5)-diazepin-1H,4H-5,7-dion, 1,3-Dimethyl-4-(dimethylaminoethyl)-8-phenyl-5,6,7,8-tetrahydropyrazolo(3,4-b)(1,5)diazepin-1H,4H-5,7-dion, 4-(2-diethylaminoethyl)-1,3-dimethyl-8-phenyl-5,6,7,8-tetrahydropyrazolo(3,4-b)(1,5)diazepin-1H,4H-5,7-dion, 1,3-Dimethyl-4-(2,2,2-trifluorethyl)-8-phenyl-5,6,7,8-tetrahydropyrazolo(3,4-b)(1,5)diazepin-1H,4H-5,7-dion, 2,3-Dimethyl-4-(2-methylsulfonylethyl)-8-phenyl-5,6,7,8-tetrahydropyrazolo-(3,4-b)(1,5)diazepin-1H,4H-5,7-dion, 1,4-Diethyl-3-methyl-8-phenyl-5,6,7,8-tetrahydropyrazolo(3,4-b)(1,5)diazepin-1H,4H-5,7-dion, 4-Allyl-1-ethyl-3-methyl-8-phenyl-5,6,7,8-tetrahydropyrazolo(3,4-b)(1,5) diazepin-1H,4H-5,7-dion, 1-Ethyl-3-methyl-4-(2-propinyl)-

BAD ORIGINAL

8-phenyl-5,6,7,8-tetrahydropyrazolo(3,4-b)(1,5)diazepin-1H,4H-5,7-dion, 4-Cyclopropylmethyl-1-ethyl-3-methyl-8-phenyl-5,6,7,8-tetrahydropyrazolo(3,4-b)(1,5)diazepin-1H,4H-5,7-dion, 1-Ethyl-3-methyl-4-(2-dimethylaminoethyl)-8-phenyl-5,6,7,8-tetrahydropyrazolo(3,4-b)(1,5)diazepin-1H,4H-5,7-dion, 1-Ethyl-4-(2-diethylaminoethyl)-3-methyl-8-phenyl-5,6,7,8-tetrahydropyrazolo(3,4-b)(1,5)diazepin-1H,4H-5,7-dion, 1-Ethyl-4-(2,2,2-trifluorethyl)-3-methyl-8-phenyl-5,6,7,8-tetrahydropyrazolo(3,4-b)(1,5)diazepin-1H,4H-5,7-dion, 1-Ethyl-3-methyl-4(2-methylsulfonylethyl)-8-phenyl-5,6,7,8-tetrahydropyrazolo(3,4-b)(1,5)diazepin-1H,4H-5,7-dion, 3-Methyl-4-ethyl-1,8-diphenyl-5,6,7,8-tetrahydropyrazolo(3,4-b)(1,5)diazepin-1H,4H-5,7-dion, 4-Allyl-3-methyl-1,8-diphenyl-5,6,7,8-tetrahydropyrazolo(3,4-b)(1,5)diazepin-1H,4H-5,7-dion, 3-Methyl-1,8-diphenyl-4-(2-propinyl)-5,6,7,8-tetrahydropyrazolo(3,4-b)(1,5)diazepin-1H,4H-5,7-dion, 4-Cyclopropylmethyl-3-methyl-1,8-diphenyl-5,6,7,8-tetrahydropyrazolo(3,4-b)(1,5)diazepin-1H,4H-5,7-dion, 3-Methyl-4-(2-dimethylaminoethyl)-1,8-diphenyl-5,6,7,8-tetrahydropyrazolo-1H,4H-5,7-dion, 4-(2-Diethylaminoethyl)-3-methyl-1,8-diphenyl-5,6,7,8-tetrahydropyrazolo(3,4-b)(1,5)diazepin-1H,4H-5,7-dion, 4-(2,2,2-Trifluorethyl)-3-methyl-1,8-diphenyl-5,6,7,8-tetrahydropyrazolo(3,4-b)(1,5)diazepin-1H,4H-5,7-dion, 3-Methyl-4-(2-methylsulfonylethyl)-1,8-diphenyl-5,6,7,8-tetrahydropyrazolo(3,4-b)(1,5)diazepin-1H,4H-5,7-dion, 1-Benzyl-4-ethyl-3-methyl-8-phenyl-5,6,7,8-tetrahydropyrazolo(3,4-b)(1,5)diazepin-1H,4H-5,7-dion, 1-Benzyl-3-methyl-8-phenyl-4-(2-propinyl)-5,6,7,8-tetrahydropyrazolo(3,4-b)(1,5)diazepin-1H,4H-5,7-dion, 4-Allyl-1-benzyl-3-methyl-8-phenyl-5,6,7,8-tetrahydropyrazolo(3,4-b)(1,5)diazepin-1H,4H-5,7-dion, 1-Benzyl-4-cyclopropylmethyl-3-methyl-8-phenyl-5,6,7,8-tetrahydropyrazolo(3,4-b)(1,5)diazepin-1H,4H-5,7-dion 1-Benzyl-3-methyl-4-(2-dimethylaminoethyl)-8-phenyl-5,6,7,8-tetrahydropyrazolo(3,4-b)(1,5)diazepin-1H,4H-5,7-dion, 1-Benzyl-4-(2-diethylaminoethyl)-3-methyl-8-phenyl-5,6,7,8-tetrahydropyrazolo(3,4-b)(1,5)diazepin-1H,4H-5,7-dion, 1-Benzyl-4-(2,2,2-trifluorethyl)-3-methyl-8-phenyl-tetrahydropyrazolo(3,4-b)(1,5)diazepin-1H,4H-5,7-dion, 1-Benzyl-3-methyl-4-(2-methylsulfonylethyl)-8-phenyl-5,6,7,8-tetrahydropyrazolo(3,4-b)(1,5)diazepin-1H,4H-5,7-dion, 1,3,4,6-Tetramethyl-8-phenyl-5,6,7,8-tetrahydropyrazolo(3,4-b)(1,5)diazepin-1H,4H-5,7-dion, 1,3,6-Trimethyl-8-phenyl-5,6,7,8-tetrahydropyrazolo(3,4-b)(1,5)-diazepin-1H,4H-5,7-dion, 1,3-Dimethyl-6-hydroxy-8-phenyl-5,6,7,8-tetrahydropyrazolo(3,4-b)(1,5)diazepin-1H,4H-5,7-dion, 1,3-Dimethyl-6-oxydimethylcarbamoyl-8-phenyl-5,6,7,8-tetrahydropyrazolo(3,4-b)(1,5)diazepin-1H,4H-5,7-dion, 6-Hydroxy-1,3,4-trimethyl-8-phenyl-5,6,7,8-tetrahydropyrazolo(3,4-b)(1,5)diazepin-1H,4H-5,7-dion, 1,3,4-trimethyl-6-oxydimethylcarbamoyl-8-phenyl-5,6,7,8-tetrahydropyrazolo(3,4-b)(1,5)diazepin-1H,4H-5,7-dion, 6-Hydroxy-1-ethyl-3-methyl-8-phenyl-5,6,7,8-tetrahydropyrazolo(3,4-b)(1,5)diazepin-1H,4H-5,7-dion, 1-Ethyl-6-hydroxy-3,4-dimethyl-8-phenyl-5,6,7,8-tetrahydropyrazolo (3,4-b)(1,5)diazepin-1H,4H-5,7-dion, 1-Ethyl-3-methyl-6-oxydimethylcarbamoyl-8-phenyl-5,6,7,8-tetrahydropyrazolo (3,4-b)(1,5)diazepin-1H,4H-5,7-dion, 1-Ethyl-3,4-dimethyl-6-oxydimethylcarbamoyl-8-phenyl-5,6,7,8-tetrahydropyrazolo(3,4-b)(1,5)diazepin-1H,4H-5,7-dion, 6-Hydroxy-3-methyl-1,8-diphenyl-5,6,7,8-tetrahydropyrazolo(3,4-b) (1,5)diazepin-1H,4H-5,7-dion, 6-Hydroxy-3,4-dimethyl-1,8-diphenyl-5,6,7,8-tetrahydropyrazolo(3,4-b)(1,5)diazepin-1H,4H-5,7-dion, 3-Methyl-6-oxydimethylcarbamoyl-1,8-diphenyl-5,6,7,8-tetrahydropyrazolo(3,4-b)(1,5)diazepin-1H,4H-5,7-dion, 3,4-Dimethyl-6-oxydimethylcarbamoyl-1,8-diphenyl-5,6,7,8-tetrahydropyrazolo(3,4-b)(1,5)diazepin-1H,4H-5,7-dion, 1-Benzyl-3,6-dimethyl-8-phenyl-5,6,7,8-tetrahydropyrazolo(3,4-b)(1,5)diazepin-1H,4H-5,7-dion, 1-Benzyl-3,4,6-trimethyl-8-phenyl-5,6,7,8-tetrahydropyrazolo(3,4-b)(1,5)diazepin-1H,4H-5,7-dion, 1-Benzyl-6-hydroxy-3-methyl-8-phenyl-5,6,7,8-tetrahydropyrazolo (3,4-b)(1,5)diazepin-1H,4H-5,7-dion, 1-Benzyl-6-hydroxy-3,4-dimethyl-8-phenyl-5,6,7,8-tetrahydropyrazolo(3,4-b)(1,5) diazepin-1H,4H-5,7-dion, 1-Benzyl-3-methyl-6-oxydimethylcarbamoyl-8-phenyl-5,6,7,8-tetrahydropyrazolo(3,4-b)(1,5) diazepin-1H,4H-5,7-dion, 1-Benzyl-3,4-dimethyl-6-oxydimethylcarbamoyl-8-phenyl-5,6,7,8-tetrahydropyrazolo (3,4-b)(1,5)diazepin-1H,4H-5,7-dion, 1-Methyl-8-(2-Chlorphenyl)-5,6,7,8-tetrahydropyrazolo(3,4-b)(1,5)diazepin-1H,4H-5,7-dion, 1,4-Dimethyl-8-(2,4-dichlorphenyl)-5,6,7,8-tetrahydropyrazolo(3,4-b)(1,5)-diazepin-1H,4H-5,7-dion, 1-Methyl-4-allyl-8-(2-pyridyl)-5,6,7,8-tetrahydropyrazolo(3,4-b)(1,5)diazepin-1H,4H-5,7-dion, 1-Methyl-4-(2-propinyl)-8-(2-trifluormethylphenyl)-5,6,7,8-tetrahydropyrazolo(3,4-b)(1,5)diazepin-1H,4H-5,7-dion, 1-Methyl-4-cyclopropylmethyl-8-(o-tolyl)-5,6,7,8-tetrahydropyrazolo(3,4-b)(1,5)diazepin-1H,4H-5,7-dion, 1-Methyl-4-(2-dimethylaminoethyl)-8-(3-chlorphenyl)-5,6,7,8-tetrahydropyrazolo(3,4-b)(1,5)diazepin-1H,4H-5,7-dion, 1-Methyl-4-(2-diethylaminoethyl)-8-(3,4-dichlorphenyl)-5,6,7,8-tetrahydropyrazolo(3,4-b)(1,5)diazepin-1H,4H-5,7-dion, 1-Methyl-4-(2,2,2-trifluorethyl)-8-(4-chlorphenyl)-5,6,7,8-tetrahydropyrazolo(3,4-b)(1,5)diazepin-1H,4H-5,7-dion, 1-Methyl-4-(2-methylsulfonylethyl)-8-(p-tolyl)-5,6,7,8-tetrahydropyrazolo(3,4-b)(1,5)diazepin-1H,4H-5,7-dion, 1,3-Dimethyl-8-(o-tolyl)-5,6,7,8-tetrahydropyrazolo(3,4-b)(1,5)diazepin-1H,4H-5,7-dion, 1,3,4-Trimethyl-8-(3-chlorphenyl)-5,6,7,8-tetrahydropyrazolo(3,4-b)(1,5)diazepin-1H,4H-5,7-dion, 4-Allyl-1,3-dimethyl-8-(3,4-dichlorphenyl)-5,6,7,8-tetrahydropyrazolo(3,4-b)(1,5)diazepin-1H,4H-5,7-dion, 1,3-Dimethyl-4-(2-propinyl)-8-(4-chlorphenyl)-5,6,7,8-tetrahydropyrazolo-(3,4-b)(1,5)diazepin-1H,4H-5,7-dion, 1,3-Dimethyl-4-cyclopropylmethyl-8-(p-tolyl)-5,6,7,8-tetrahydropyrazolo(3,4-b)(1,5)diazepin-1H,4H-5,7-dion, 1,3-Dimethyl-4-(2-dimethylaminoethyl)-8-(2-chlorphenyl)-5,6,7,8-tetrahydropyrazolo-(3,4-b)(1,5)diazepin-1H,4H-5,7-dion, 1,3-Dimethyl-4-(2-diethylaminoethyl)-8-(2,4-dichlorphenyl)-5,6,7,8-tetrahydropyrazolo(3,4-b)(1,5)diazepin-1H,4H-5,7-dion, 1,3-Dimethyl-4-(2,2,2-trifluorethyl)-8-(2-cyanophenyl)-5,6,7,8-tetrahydropyrazolo(3,4-b)(1,5)diazepin-1H,4H-5,7-dion, 1,3-Dimethyl-4-(2-methylsulfonylethyl)-8-(2-trifluormethylphenyl)-5,6,7,8-tetrahyropyrazolo(3,4-b)(1,5)diazepin-1H,4H-5,7-dion, 1-Ethyl-3,4-dimethyl-8-(2-chlorphenyl)-5,6,7,8-tetrahydropyrazolo(3,4-b)(1,5)diazepin-1H,4H-5,7-dion, 4-Allyl-1-ethyl-3-methyl-8-(2,4-dichlorphenyl)-5,6,7,8-tetrahydropyrazolo (3,4-b)(1,5)diazepin-1H,4H-5,7-dion, 1-Ethyl-3-methyl-4-(2-propinyl)-8-(2-pyridyl)-5,6,7,8-tetrahydropyrazolo(3,4-b)(1,5)diazepin-1H,4H-5,7-dion, 4-Cyclopropylmethyl-1-ethyl-3-methyl-8-(2-trifluormethylphenyl)-5,6,7,8-tetrahydropyrazolo(3,4-b)(1,5)diazepin-1H,4H-5,7-dion, 1-Ethyl-3-methyl-4-(2-dimethylaminoethyl)-8-(o-tolyl)-5,6,7,8-tetrahydropyrazolo(3,4-b)(1,5)diazepin-1H,4H-5,7-dion, 1-Ethyl-3-methyl-4-(2-methylsulfonylethyl)-8-(4-chlorphenyl)-5,6,7,8-tetrahydropyrazolo(3,4-b)(1,5)diazepin-1H,4H-5,7-dion, 1-Phenyl-3,4-dimethyl-8-(m-tolyl)-5,6,7,8-tetrahydropyrazolo(3,4-b)(1,5)-diazepin-1H,4H-5,7-dion, 4-Allyl-3-methyl-1-phenyl-8-(3,4-dichlorphenyl)-5,6,7,8-tetrahydropyrazolo(3,4-b)(1,5)-diazepin-1H,4H-5,7-dion, 3-Methyl-1-phenyl-4-(2-propinyl)-8-(3-chlorphenyl)-5,6,7,8-tetrahydropyrazolo(3,4-b)(1,5)diazepin-1H,4H-5,7-dion, 4-Cyclopropylmethyl-3-methyl-1-phenyl-8-(2-cyanophenyl)-5,6,7,8-tetrahydropyrazolo(3,4-b)(1,5)diazepin-1H,4H-5,7-dion, 3-Methyl-4-(2-dimethylaminoethyl)-1-phenyl-8-(2-pyridyl)-5,6,7,8-tetrahydropyrazolo-(3,4-b)(1,5)diazepin-1H,4H-5,7-dion, 4-(2-Diethylaminoethyl)-3-methyl-1-phenyl-8-(2-chlorphenyl)-5,6,7,8-tetrahydropyrazolo(3,4-b)(1,5)diazepin-1H,4H-5,7-dion, 4-(2,2,2-Trifluorethyl)-3-methyl-1-phenyl-8-(o-tolyl)-5,6,7,8-tetrahydropyrazolo(3,4-b)(1,5)diazepin-1H,4H-5,7-dion, 3-Methyl-1-phenyl-4-(2-methylsulfonylethyl)-8-(2,4-dichlorphenyl)-5,6,7,8-tetrahydropyrazolo(3,4-b)(1,5)diazepin-1H,4H-5,7-dion, 1-Benzyl-3,4-dimethyl-8-(2-chlorphenyl)-5,6,7,8-tetrahydropyrazolo(3,4-b)(1,5)diazepin-1H,4H-5,7-dion, 4-Allyl-1-benzyl-3-methyl-8-(3,4-dichlorphenyl)-5,6,7,8-tetrahydro-

BAD ORIGINAL

pyrazolo(3,4-b)(1,5)diazepin-1H,4H-5,7-dion, 1-Benzyl-3-methyl-4-(2-propinyl)-8-(o-tolyl)-5,6,7,8-tetrahydropyrazolo(3,4-b)(1,5)diazepin-1H,4H-5,7-dion, 1-Benzyl-4-cyclopropylmethyl-3-methyl-8-(4-chlorphenyl)-5,6,7,8-tetrahydropyrazolo(3,4-b)(1,5)diazepin-1H,4H-5,7-dion, 1-Benzyl-3-methyl-4-(2-dimethylaminoethyl)-8-(3-chlorphenyl)-5,6,7,8-tetrahydropyrazolo(3,4-b)(1,5)diazepin-1H,4H-5,7-dion, 1-Benzyl-3-methyl-4-(2-diethylaminoethyl)-8-(2-trifluormethylphenyl)-5,6,7,8-tetrahydropyrazolo(3,4-b)(1,5)diazepin-1H,4H-5,7-dion, 1-Benzyl-3-methyl-4-(2,2,2-trifluorethyl)-8-(4-chlorphenyl)-5,6,7,8-tetrahydropyrazolo(3,4-b)(1,5)diazepin-1H,4H-5,7-dion, 1-Benzyl-3-methyl-4-(2-methylsulfonylethyl)-8-(2-cyanophenyl)-5,6,7,8-tetrahydropyrazolo(3,4-b)(1,5)diazepin-1H,4H-5,7-dion, 1,3,6-Trimethyl-8-(2-trifluormethylphenyl)-5,6,7,8-tetrahydropyrazolo(3,4-b)(1,5)diazepin-1H,4H-5,7-dion, 1,3,4,6-Tetramethyl-8-(o-tolyl)-5,6,7,8-tetrahydropyrazolo(3,4-b)(1,5)-diazepin-1H,4H-5,7-dion, 1,3-Dimethyl-6-hydroxy-8-(2,4-dichlorphenyl)-5,6,7,8-tetrahydropyrazolo(3,4-b)(1,5)diazepin-1H,4H-5,7-dion, 1,3,4-Trimethyl-6-hydroxy-8-(2-pyridyl)-5,6,7,8-tetrahydropyrazolo(3,4-b)(1,5)diazepin-1H,4H-5,7-dion, 1,3-Dimethyl-6-oxydimethylcarbamoyl-8-(2-chlorphenyl)-5,6,7,8-tetrahydropyrazolo(3,4-b)(1,5) diazepin-1H,4H-5,7-dion, 1,3,4-Trimethyl-6-oxydimethylcarbamoyl-8-(m-tolyl)-5,6,7,8-tetrahydropyrazolo(3,4-b)(1,5)diazepin-1H,4H-5,7-dion, 1-Ethyl-3-methyl-6-hydroxy-8-(2,4-dichlorphenyl)-5,6,7,8-tetrahydropyrazolo(3,4-b)(1,5)diazepin-1H,4H-5,7-dion, 1-Ethyl-3,4-dimethyl-6-hydroxy-8-(2-cyanophenyl)-5,6,7,8-tetrahydropyrazolo(3,4-b)(1,5)diazepin-1H,4H-5,7-dion, 1-Ethyl-3-methyl-6-oxydimethylcarbamoyl-8-(2-pyridyl)-5,6,7,8-tetrahydropyrazolo(3,4-b)(1,5)diazepin-1H,4H-5,7-dion, 1-Ethyl-3,4-dimethyl-6-oxydimethylcarbamoyl-8-(3-chlorphenyl)-5,6,7,8-tetrahydropyrazolo(3,4-b)(1,5)diazepin-1H,4H-5,7-dion, 1-Ethyl-3,6-dimethyl-8-(4-chlorphenyl)-5,6,7,8-tetrahydropyrazolo(3,4-b)(1,5)diazepin-1H,4H-5,7-dion, 1-Ethyl-3,4,6-trimethyl-8-(m-tolyl)-5,6,7,8-tetra-

BAD ORIGINAL

hydropyrazolo(3,4-b)(1,5)diazepin-1H,4H-5,7-dion, 3,6-Dimethyl-1-phenyl-8-(3,4-dichlorphenyl)-5,6,7,8-tetrahydropyrazolo(3,4-b)(1,5)diazepin-1H,4H-5,7-dion, 3,4,6-Trimethyl-1-phenyl-8-(2-trifluormethylphenyl)-5,6,7,8-tetrahydropyrazolo(3,4-b)(1,5)diazepin-1H,4H-5,7-dion, 3-Methyl-1-phenyl-6-hydroxy-8-(2-cyanophenyl)-5,6,7,8-tetrahydropyrazolo(3,4-b)(1,5)diazepin-1H,4H-5,7-dion, 3,4-Dimethyl-1-phenyl-6-oxydimethylcarbamoyl-8-(2-bromphenyl)-5,6,7,8-tetrahydropyrazolo(3,4-b)(1,5)diazepin-1H,4H-5,7-dion, 3,4-Dimethyl-1-phenyl-6-hydroxy-8-(3-bromphenyl)-5,6,7,8-tetrahydropyrazolo(3,4-b)(1,5)diazepin-1H,4H-5,7-dion, 3-Methyl-1-phenyl-6-oxydimethylcarbamoyl-8-(4-bromphenyl)-5,6,7,8-tetrahydropyrazolo(3,4-b)(1,5)diazepin-1H,4H-5,7-dion, 1-Benzyl-3,6-dimethyl-8-(2-pyridyl)-5,6,7,8-tetrahydropyrazolo(3,4-b)(1,5)diazepin-1H,4H-5,7-dion, 1-Benzyl-3,4,6-trimethyl-8-(o-tolyl)-5,6,7,8-tetrahydropyrazolo-(3,4-b)(1,5)diazepin-1H,4H-5,7-dion, 1-Benzyl-6-hydroxy-3-methyl-8-(2-trifluormethyl)-5,6,7,8-tetrahydropyrazolo-(3,4-b)(1,5)diazepin-1H,4H-5,7-dion, 1-Benzyl-6-hydroxy-3,4-dimethyl-8-(2,4-dichlorphenyl)-5,6,7,8-tetrahydropyrazolo-(3,4-b)(1,5)diazepin-1H,4H-5,7-dion, 1-Benzyl-3-methyl-6-oxydimethylcarbamoyl-8-(2-cyanophenyl)-5,6,7,8-tetrahydropyrazolo(3,4-b)(1,5)diazepin-1H,4H-5,7-dion, 1-Benzyl-3,4-dimethyl-6-oxydimethylcarbamoyl-8-(3,4-dichlorphenyl)-5,6,7,8-tetrahydropyrazolo(3,4-b)(1,5)diazepin-1H,4H-5,7-dion.

Gegenstand der Erfindung sind weiterhin Verfahren zur Herstellung dieser Verbindungen sowie pharmazeutische Zubereitungen dieser Verbindungen und ihre Verwendung als Arzneimittel.

Die Herstellung kann in an sich bekannter Weise dadurch erfolgen, daß man

a) eine Verbindung der Formel

BAD ORIGINAL

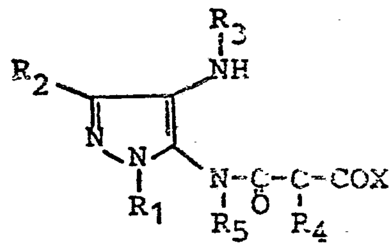

II

wobei $R_1$, $R_2$, $R_3$, $R_4$ und $R_5$ die oben angegebenen Bedeutungen haben und X eine Hydroxygruppe, eine Mercaptogruppe, ein Halogenatom, eine Alkoxygruppe, eine Alkylmercaptogruppe, eine Aminogruppe, eine Alkylaminogruppe, eine Dialkylaminogruppe, eine Benzoyloxygruppe, eine Aryloxygruppe, eine Acyloxygruppe oder die Gruppe $N_3$ bedeutet, zum Siebenring zyklisiert; oder

b) eine Verbindung der Formel

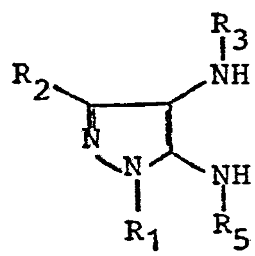

III

wobei $R_1$, $R_2$, $R_3$ und $R_5$ die oben angegebene Bedeutung haben, mit einem aktivierten Malonsäurederivat, z.B. Malonsäuredihalogenid, Malonester, Kohlensuboxyd, Meldrumsäure oder mit Malonsäure selbst zum Siebenring kondensiert; oder

c) eine Verbindung der Formel

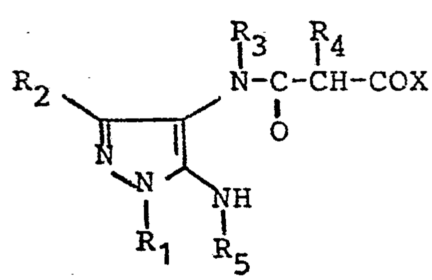

IV

wobei $R_1$, $R_2$, $R_3$, $R_4$ und $R_5$ die oben angegebene Bedeutung haben und für X die unter a) angegebenen Gruppen in Frage kommen, mit den unter a) beschriebenen Verfahren zum Siebenring zyklisiert, und gegebenenfalls in den erhaltenen Verbindungen, in denen einer oder beide der Reste $R_3$ und $R_4$ Wasserstoff sind, in an sich bekannter Weise diese Positionen gleichzeitig oder nacheinander durch Alkylierung oder Acylierung substituiert.

Das Verfahren a) kann mit oder ohne Lösungsmittel durch Erhitzen auf 50-250°C, gegebenenfalls unter Zusatz eines für derartige Reaktionen üblichen Kondensationsmittels, durchgeführt werden. Als Lösungsmittel kommen z.B. in Frage: Aliphatische Alkohole (Methanol, Ethanol), Dioxan, Dimethylformamid, Benzol, Toluol, wäßriger oder alkoholischer Salzsäure, Eisessig, Polyphosphorsäure, $H_2SO_4$, wobei die drei letzteren Kondensationsmittel darstellen, die auch in den genannten Lösungsmitteln verwandt werden können. Als Kondensationsmittel kommen sonst noch in Betracht: Metall-, insbesondere Alkalialkoholate, Alkaliamide, Alkalihydride (NaH), starke Säuren wie Trifluoressigsäure, p-Toluolsulfonsäure, aber auch Dehydratisierungsmittel wie Dicyclohexylcarbodiimid etc.. Bei den Halogenatomen, die bei dem Verfahren a) in Betracht kommen, handelt es sich vorzugsweise um Cl, Br oder auch die halogenähnliche Gruppe $N_3$. Falls X eine Alkoxygruppe, eine Alkylmercaptogruppe, eine Alkylaminogruppe oder eine Dialkylaminogruppe ist, handelt es sich im allgemeinen um solche mit Alkylresten mit 1 bis 6 C-Atomen. Bedeutet X eine Acyloxygruppe, so handelt es sich vorzugsweise um eine aliphatische Acylgruppe aus 2 bis 6 C-Atomen. Bei einer Aryloxygruppe handelt es sich bevorzugt um eine Phenoxygruppe.

Das Verfahren b) ist dadurch gekennzeichnet, daß man das aktivierte Malonsäurederivat unter Verwendung eines geeigneten inerten Lösungsmittels wie z.B. Benzol, Toluol,

BAD ORIGINAL

Xylol, Ether, Tetrahydrofuran, Dioxan oder Dimethylformamid bei Raumtemperatur oder vorteilhafter bei der Siedetemperatur des jeweiligen Lösungsmittels umsetzt. In manchen Fällen erweist sich auch der Zusatz einer tertiären organischen Base wie z.B. Pyridin, Triethylamin etc. als günstig für den Ablauf der Reaktion. Die Umsetzung mit Malonsäure erfolgt am besten unter Verwendung von starken Säuren wie z.B. HCl, $H_2SO_4$, Trifluoressigsäure, Polyphosphorsäure etc..

Das Verfahren c) ist dadurch gekennzeichnet, daß die Zyklisierung zum Siebenring unter den gleichen Bedingungen erfolgt wie für Verfahren a) beschrieben.

Die Reste $R_3$ und $R_4$ können auch nachträglich durch Alkylierung bzw. Acylierung nach bekannten Methoden eingeführt werden. Beispielsweise können Verbindungen der Formel I, bei denen $R_3$ und $R_4$ Wasserstoffatome sind, mit Protonenakzeptoren wie z.B. Natriumhydrid, Natriumamid, Kalium-tert.Butylat oder feinverteiltem Natrium in einem inerten Lösungsmittel in ein Monoalkalisalz überführt werden, das dann in an sich bekannter Weise alkyliert oder acyliert werden kann. Als Alkylierungsmittel kommen beispielsweise Alkylhalogenide der Formel RHal oder Ester der Formel $ArSO_2OR$ und $SO_2(OR)_2$ in Betracht, wobei Hal ein Halogenatom (insbesondere Cl, Br, J) und Ar ein aromatischer Rest wie z.B. Phenyl oder ein durch ein oder mehrere niedere Alkylreste substituierter Phenylrest ist. Bei R handelt es sich dann jeweils um die unter $R_3$ (außer H) aufgeführten Gruppen. Die Acylierung kann unter den gleichen Bedingungen erfolgen. Als Acylierungsmittel kommen beispielsweise in Betracht: Ketene sowie Säurehalogenide, Säureanhydride, oder Säureester von aliphatischen Carbonsäuren mit 2 bis 6 C-Atomen bzw. von Kohlensäurehalbesterhalogeniden mit 1 bis 6 C-Atomen.

Die für die Verfahren a), b) und c) verwendeten Ausgangsmaterialien können beispielsweise auf folgenden Wegen

BAD ORIGINAL

erhalten werden:

a) Eine Verbindung der Formel

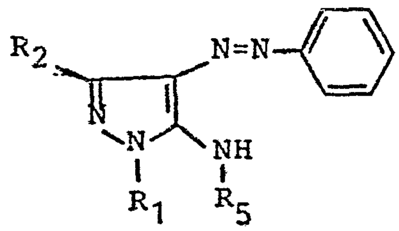

V

worin $R_1$, $R_2$ und $R_5$ die oben angegebene Bedeutung haben, wird umgesetzt mit einer Verbindung der Formel

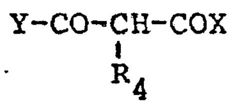

VI

wobei $R_4$ und X die oben angegebene Bedeutung haben und Y ein Chlor- oder Bromatom, eine Azidogruppe ($N_3$) oder eine Alkyloxy- oder Aryloxygruppe bedeutet, in einem inerten Lösungsmittel wie Dioxan, Tetrahydrofuran, Chloroform, Benzol oder Toluol bei Temperaturen zwischen 0 und 200°C mit oder ohne Zusatz eines Säureakzeptors zu einer Verbindung der Formel

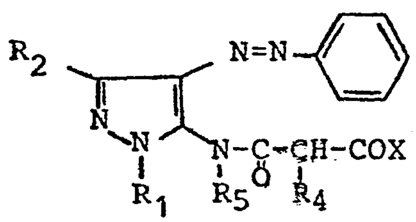

VII

Bei dieser Verbindung wird dann die Azogruppe reduktiv gespalten, wobei die Aminoverbindung der Formel II entsteht, in der $R_3$ Wasserstoff bedeutet. Der Rest $R_3$ kann dann beispielsweise durch Alkylierung oder Acylierung nach den bekannten Verfahren eingeführt werden. Die reduktive Spaltung der Azogruppe kann erfolgen durch katalytische Hydrierung (mit Pd, Pt, Raney-Nickel in Alkoholen, Dioxan, Tetrahydrofuran bei 0-60°C und bei 1-50 Atm. $H_2$-Druck) oder durch chemische Reduktion z.B. mit Natriumdithionit in

BAD ORIGINAL

wäßriger oder alkoholischer Lösung, mit $SnCl_2$ in HCl oder mit Zink in Eisessig oder neutraler, saurer oder alkalischer wäßriger Lösung.

Die Ausgangsmaterialien der Formel V sind auf dem von F.A. Amer, A.H. Harhash und M.L. Awad (Z. Naturforsch. 33 b, 660-662 (1978)) beschriebenen Wege zugänglich oder lassen sich wie folgt synthetisieren:

Ein Pyrazolon der Formel

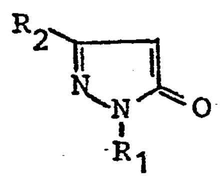

VIII

wird umgesetzt mit Benzoldiazoniumchlorid in Eisessig bei 0-5°C zum Phenylhydrazon der Formel

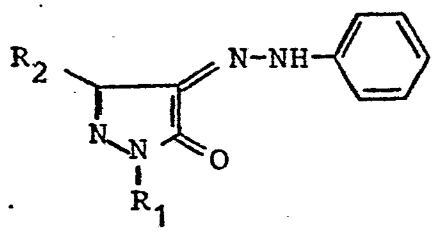

IX

das dann in siedendem $POCl_3$ in 5-Position chloriert werden kann zum 4-Benzolazo-5-chlorpyrazol der Formel

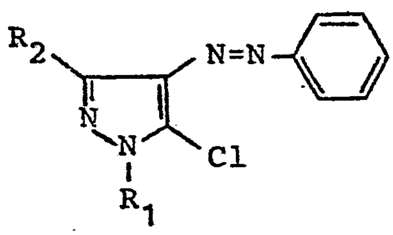

X

Anschließend wird der Chlorrest ausgetauscht gegen ein entsprechend substituiertes Anilin oder Pyridin bei 100 - 160°C mit oder ohne Lösungsmittel, wodurch die Verbindung

der Formel V entsteht.

Verbindungen der allgemeinen Formel II können außerdem auch auf folgendem Wege erhalten werden:

Eine Verbindung der Formel X

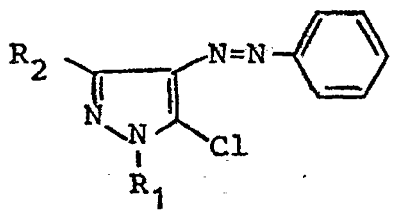

(Synthese siehe oben)
wird mit einer Verbindung der Formel

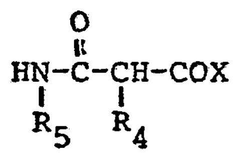

XI

wobei $R_4$ und $R_5$ die angegebenen Bedeutungen haben und für X die angegebenen Reste mit Ausnahme von Halogen, $NH_2$, Alkylamino und Azido ($N_3$) in Frage kommen, zu einer Verbindung der Formel VII umgesetzt. Man arbeitet dabei im allgemeinen in einem inerten Lösungsmittel wie z.B. Dioxan, Tetrahydrofuran, Dimethylformamid unter Zusatz eines Protonenakzeptors wie NaH, Natriumamid oder feinverteiltem Natrium. Die Temperaturen der Reaktion liegen zwischen 0 und 150°C.

Verbindungen der Formel II, in denen X = Halogen ist, können z.B. aus Verbindungen der Formel II, in denen X = OH ist, durch Umsetzung mit Halogenierungsmitteln wie Thionylchlorid oder Phosphorpentachlorid bei Temperaturen zwischen 0 bis 100°C in inerten Lösungsmitteln wie Benzol, Toluol, Dioxan oder Tetrahydrofuran erhalten werden. Die Carbonsäuren der Formel II (X = OH) kann man beispielsweise auch aus dem entsprechendem Ester (X = Alkyloxy-, Aryloxy- oder Benzyloxy-) erhalten, indem dieser unter

BAD ORIGINAL

milden Bedingungen in die Säure überführt wird, z.B. durch Hydrierung (bei X = Benzyloxy) oder durch sehr milde Hydrolyse (bei X = Aryloxy- oder verzweigter Alkyloxyrest wie tert. Butoxy-). Verbindungen der Formel II, in denen X eine Acyloxygruppe ist kann man auch aus den entsprechenden Halogeniden durch Umsetzung mit den entsprechenden Metallsalzen von Carbonsäuren, z.B. mit Natriumacetat oder Silberbenzoat in inerten Lösungsmitteln wie Aceton, Dioxan, Ether bei Temperaturen zwischen -20 und 100°C erhalten.

Verbindungen der Formel II, in denen X eine Azidogruppe ist, lassen sich beispielsweise aus den entsprechenden Halogeniden durch Umsetzung mit Alkaliaziden in inerten Lösungsmittel wie Aceton, Dioxan, Dimethylsulfoxid bei Temperaturen zwischen 0 und 100°C erhalten, oder aus Estern (X = O-Alkyl bzw. O-Aryl) durch Umsetzung mit Hydrazin, gegebenenfalls in einem inerten Lösungsmittel wie Ethanol, Dioxan, Tetrahydrofuran bei 0 bis 100°C und anschließender Reaktion des Hydrazides mit salpetriger Säure oder nitrosen Gasen in inerten Lösungsmitteln wie Alkoholen, Dioxan, Dimethylformamid bei Temperaturen zwischen 0 und 50°C.

Die Ausgangsstoffe der Formel XI können wie folgt hergestellt werden:

Ein Amin $R_5$-$NH_2$, wobei $R_5$ die obengenannte Bedeutung hat, wird mit einer Verbindung der Formel VI, worin $R_4$ und X die obengenannten Bedeutungen haben (ausgenommen Halogen, $NH_2$, Alkylamino und Azido bei X) und Y ein Chlor- oder Bromatom, eine Azidogruppe oder eine Alkyloxy- oder Aryloxygruppe bedeutet, in einem inerten Lösungsmittel wie Dioxan, Tetrahydrofuran, Chloroform, Aceton oder auch in einem Überschuß der Verbindung VI bei Temperaturen zwischen 0 und 200°C umgesetzt. Es kann hierbei beispielsweise analog Chem. Ber. 17, 739 ff (1884) oder J. Indian Chem. Soc. 37, 591 - 593 (1960) verfahren werden.

b) Bei der Verbindung der allgemeinen Formel V wird die Azogruppe unter den oben angegebenen Bedingungen reduktiv gespalten zu einer Verbindung der allgemeinen Formel III, wobei $R_3$ Wasserstoff bedeutet. Einführung des Restes $R_3$ kann im Falle einer Alkylgruppe analog zu den Angaben in Chem. Ber. 34, 4204 (1902) und 37, 552 (1904) durch Ringschluß mit Ameisensäure zum Pyrazoloimidazol, Alkylierung am Imidazol-Stickstoffatom mittels Alkyljodid und anschließende Aufspaltung des Imidazolringes mit Alkali erfolgen. Im Falle einer Acylierung läßt sich das Diamin III unter milden Bedingungen umsetzen beispielsweise mit Carbonsäurehalogeniden, -anhydriden oder Estern, wobei die primäre Aminogruppe selektiv abreagiert. Die Umsetzung erfolgt vorzugsweise in inerten Lösungsmitteln wie Ether, Dioxan, Tetrahydrofuran, Chloroform mit oder ohne Zusatz eines Protonenakzeptors bei 0 bis 100°C.

c) Verbindungen der allgemeinen Formel IV sind zugänglich durch Umsetzung von Verbindungen der allgemeinen Formel III mit Verbindungen der allgemeinen Formel VI, wobei die Aminogruppe in 4-Stellung des Pyrazolringes selektiv abreagiert. Die Reaktion kann erfolgen in einem inerten Lösungsmittel wie z.B. Benzol, Toluol, Dioxan, Tetrahydrofuran, Chloroform mit oder ohne Zusatz eines Säureakzeptors zwischen 0 und 150°C.

Die erfindungsgemäßen Verbindungen sind zur Herstellung von Arzneimitteln geeignet. Die Arzneimittel können eine oder mehrere der erfindungsgemäßen Verbindungen oder auch Mischungen derselben mit anderen pharmazeutisch wirksamen Stoffen enthalten. Zur Herstellung der Arzneimittel können die üblichen pharmazeutischen Träger und Hilfsstoffe und an sich bekannte galenische Verfahren verwendet werden. Die Arzneimittel können enteral, parenteral, oral oder perlingual angewendet werden. Beispielsweise kann die Verabreichung in Form von Tabletten, Kapseln, Pillen, Dragees, Zäpfchen, Gelees, Cremes, Puder, Liquida, Stäubepulver

BAD ORIGINAL

oder Aerosolen erfolgen. Als Liquida kommen zum Beispiel in Frage: ölige oder wässrige Lösungen oder Suspensionen, Emulsionen, injizierbare wässrige Lösungen oder Suspensionen.

Die erfindungsgemässen Verbindungen besitzen hervorragende anxiolytische Eigenschaften. Als Indikationen kommen deshalb Schlaflosigkeit, Erregung und vegetative Verstimmung in Betracht.

Die pharmazeutischen Zubereitungen enthalten im allgemeinen zwischen 1 bis 10 % der erfindungsgemässen aktiven Komponente(n).

Die anxiolytische Wirkung ist von einer sehr geringen Sedierung und guten Verträglichkeit begleitet ($LD_{50} \gg$ 1200 mg/kg p.o. an der Maus). Dies geht aus Untersuchungen hervor, bei denen der Einfluss der erfindungsgemässen Verbindungen auf die motorische Aktivität, die Hexobarbital-Narkose und den Cardiazolkrampf bei Mäusen gemessen wurde. Ausserdem wurde noch der Hamster-Zähmungstest sowie der Geller-Anxiolysetest an Ratten herangezogen.

Die niedrigste, bereits wirksame Dosis beträgt 5 mg/kg oral, 2,5 mg/kg sublingual, 1 mg/kg intravenös. Im allgemeinen werden einem Erwachsenen pro Tag 5 bis 50 mg/kg oral, 2,5 bis 25 mg/kg sublingual oder 1 bis 10 mg/kg intravenös verabreicht.

Dementsprechend können einem Erwachsenen 3 mal täglich 1 bis 3 Tabletten mit einem Gehalt von 10 bis 100 mg wirksamer Substanz oder bei intravenöser Injektion 1 bis 3 mal täglich eine Ampulle von 2 bis 4 ml Inhalt mit 0,5 bis 5 mg Substanz empfohlen werden.

Beispiel 1:

1-Ethyl-3-methyl-8-phenyl-5,6,7,8-tetrahydropyrazolo(3,4-b)-(1,5)diazepin-1H,4H-5,7-dion.

a) 4-Amino-5-anilino-1-ethyl-3-methylpyrazol

30,5 g (0,1 Mol) 5-Anilino-4-benzolazo-1-ethyl-3-methyl-pyrazol werden in 250 ml Ethanol mit 60 g Raney-Nickel bei Raumtemperatur und Normaldruck hydriert. Nach Ende der Wasserstoffaufnahme wird vom Katalysator abgesaugt und die Reaktionslösung im Vakuum eingedampft. Der Rückstand wird mit Petroläther versetzt und der Niederschlag abgesaugt. Umkristallisation aus Chloroform ergibt das analysenreine Produkt.

b) 5-Anilino-4-α-carbethoxyacetylamino-1-ethyl-3-methyl-pyrazol

2,2 g 4-Amino-5-anilino-1-ethyl-3-methylpyrazol (0,01 Mol) wurden in 20 ml Toluol gelöst, und bei Raumtemperatur wurde langsam 1 ml Malonsäuremonoethylesterchlorid (0,012 Mol) zugetropft und eine Stunde bei Raumtemperatur nachgerührt. Das Toluol wurde im Vakuum abgezogen, der Rückstand in Chloroform aufgenommen, mit eiskalter $NaHCO_3$-Lösung geschüttelt, mit Wasser gewaschen und mit $Na_2SO_4$ getrocknet. Nach Verdampfen des Lösungsmittels blieb ein gelbliches Öl zurück, das nicht kristallisierte.

c) 1-Ethyl-3-methyl-8-phenyl-5,6,7,8-tetrahydropyrazol (3,4-b)(1,5)diazepin-1H,4H-5,7-dion

3,3 g 5-Anilino-4-α-carbethoxyacetylamino-1-ethyl-3-methylpyrazol (0,01 Mol), gelöst in 100 ml Ethanol, werden mit 15 ml einer 1-molaren Natriumethanolat-Lösung versetzt und 8 Stunden bei Raumtemperatur gerührt. Anschließend wird mit HCl neutralisiert, im Vakuum eingedampft, der Rückstand mit Ether verrührt und abgesaugt. Umristallisation aus Isopropanol ergibt das analysenreine Produkt.

Fp.: 196 - 197°C.

BAD ORIGINAL

Beispiel 2:

1-Ethyl-3,4-dimethyl-8-phenyl-5,6,7,8-tetrahydropyrazolo(3,4-b)(1,5)diazepin-1H,4H-5,7-dion.

2,84 g 1-Ethyl-3-methyl-8-phenyl-5,6,7,8-tetrahydropyrazolo(3,4-b)(1,5)diazepin-1H,4H-5,7-dion (0,01 Mol) werden in 35 ml DMF gelöst und bei Raumtemperatur unter Stickstoffatmosphäre werden 0,57 g 80 %iges NaH (0,01 Mol) hinzugegeben. Nach einstündigem Rühren wird das Lösungsmittel im Vakuum abgezogen, der Rückstand in Chloroform aufgenommen, mit Wasser gewaschen und getrocknet mit $Na_2SO_4$. Das Chloroform wird eingedampft und das Produkt aus Isopropanol/Wasser umkristallisiert.
Fp.: 125 - 126°C

Beispiel 3:

1,3-Dimethyl-8-phenyl-5,6,7,8-tetrahydropyrazolo(3,4-b)(1,5)diazepin-1H,4H-5,7-dion.

Unter Eiskühlung wird zu einer Lösung von 280 mg Kohlensuboxid (Darstellung siehe H. Staudinger, S. Bereza, Ber. 41, 4461 (1908)) (0,04 Mol) in 70 ml Ether eine Lösung von 500 mg 4-Amino-5-anilino-1,3-dimethylpyrazol (0,004 Mol) langsam zugetropft. Anschließend wird noch eine Stunde bei 0°C gerührt und der Niederschlag abgesaugt. Umkristallisation aus Isopropanol ergibt das Produkt.
Fp.: 249°C

Beispiel 4:

3-Methyl-1,8-diphenyl-5,6,7,8-tetrahydropyrazolo(3,4-b)-(1,5)diazepin-1H,4H-5,7-dion.

a) 4-Benzolazo-5-(N-α-methoxycarbonylacetylanilino)-3-methyl-1-phenylpyrazol

1) Zu einer Lösung von 19,3 g (0,1 Mol) Malonsäuremethylestermonoanilid in 100 ml DMF unter Stickstoffatmosphäre gibt man bei Raumtemperatur 2,4 g NaH (0,1 Mol) hinzu und rührt 30 Min. nach. Dann wird unter Kühlung eine Lösung von 30 g (0,1 Mol) 4-Benzolazo-5-chlor-3-methyl-1-phenylpyrazol in 50 ml DMF langsam zugetropft und eine Stunde auf 50°C erwärmt. Man setzt 10 ml Ethanol hinzu und 5 ml Eisessig und dampft die Lösung im Vakuum ein. Der Rückstand wird in Chloroform aufgenommen, mit Wasser gewaschen, mit $Na_2SO_4$ getrocknet und eingedampft. Umkristallisation aus Petrolether liefert gelbe Kristalle vom Fp.: 158 - 159°C

2) 3,5 g (0,01 Mol) 5-Anilino-4-benzolazo-3-methyl-1-phenylpyrazol werden mit 1,36 ml (0,012 Mol) Malonsäuremonomethylesterchlorid in 50 ml siedendem Benzol umgesetzt, bis die HCl-Entwicklung beendet ist. Dann wird das Benzol im Vakuum abgezogen, der Rückstand in $CHCl_3$ aufgenommen, mit kalter $NaHCO_3$-Lösung und Wasser gewaschen, getrocknet und eingedampft. Umkristallisation aus Petrolether liefert gelbe Kristalle identisch mit der unter 4 a)$_{1)}$ beschriebenen Verbindung.

b) 4-Amino-5-(N-α-methoxycarbonylacetylanilino)-3-methyl-1-phenylpyrazol

4,5 g (0,01 Mol) 4-Benzolazo-5-(N-α-methoxycarbonylacetylanilino)-3-methyl-1-phenylpyrazol, gelöst in 100 ml Ethanol, werden mit 10 g Raney-Nickel bei Raumtemperatur und einer Atmosphäre Wasserstoffdruck hydriert. Nach Ende der Wasserstoffaufnahme wird vom Katalysator abgesaugt und die Reaktionslösung im Vakuum eingedampft. Als Rückstand bleibt ein farbloses Öl, das sich nicht kristallisieren läßt.

c) 3-Methyl-1,8-diphenyl-5,6,7,8-tetrahydropyrazolo-(3,4-b)(1,5)diazepin-1H,4H-5,7-dion

3,6 g (0,01 Mol) des nach Verfahren 4 b) erhaltenen Öls werden in 100 ml Ethanol gelöst. Die Lösung wird nach

BAD ORIGINAL

Zugabe von 1 ml konz. HCl solange am Rückfluß gekocht, bis das Ausgangsmaterial verschwunden ist (DC-Kontrolle). Anschließend wird die Lösung im Vakuum eingedampft, der Rückstand in Chloroform aufgenommen, mit $NaHCO_3$-Lösung und Wasser gewaschen, getrocknet und eingedampft. Umkristallisation aus Isopropanol liefert weiße Kristalle. Fp.: 262°C.

Nach den beschriebenen Vorschriften wurden außerdem folgende Verbindungen hergestellt:

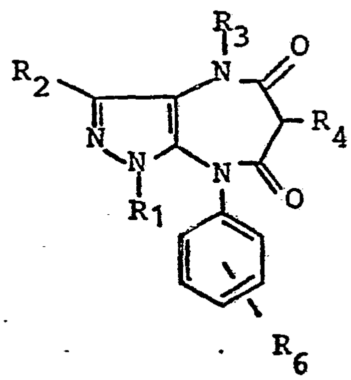

| Bsp. | $R_1$ | $R_2$ | $R_3$ | $R_4$ | $R_6$ | Fp (°C) | hergestellt gemäß Bsp. |
|---|---|---|---|---|---|---|---|
| 5 | $-C_2H_5$ | $-CH_3$ | H | H | 2-$CH_3$ | 221 | 4 |
| 6 | $-C_2H_5$ | $-CH_3$ | H | H | 2-Cl | 206-207 | 4 |
| 7 | $-CH_3$ | $-CH_3$ | H | H | 4-$CH_3$ | 251 | 4 |
| 8 | $-C_2H_5$ | $-CH_3$ | H | H | 4-Cl | 186 | 4 |
| 9 | $-C_2H_5$ | $-CH_3$ | H | H | 4-$CH_3$ | 183-184 | 4 |
| 10 | $-CH_3$ | $-CH_3$ | H | H | 4-Cl | 261-262 | 4 |
| 11 | $-CH_3$ | $-CH_3$ | H | H | 2-$CH_3$ | 235 | 4 |
| 12 | $-CH_3$ | $-CH_3$ | H | H | 2-Cl | 228 | 4 |
| 13 | 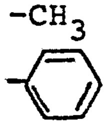 | $-CH_3$ | H | H | 4-$CH_3$ | 245-246 | 4 |
| 14 | $-CH_3$ | $-CH_3$ | $-CH_3$ | H | 4-$CH_3$ | 165 | 2 |
| 15 | $-CH_3$ | $-CH_3$ | $-CH_3$ | H | 2-$CH_3$ | 163 | 2 |
| 16 | $-CH_3$ | $-CH_3$ | $-CH_3$ | H | 4-Cl | 165-166 | 2 |
| 17 | $-C_2H_5$ | $-CH_3$ | $-CH_3$ | H | 2-Cl | 181-182 | 2 |

BAD ORIGINAL

| Bsp. | $R_1$ | $R_2$ | $R_3$ | $R_4$ | $R_6$ | Fp. (°C) | hergestellt gemäß Bsp. |
|---|---|---|---|---|---|---|---|
| 18 | $-C_2H_5$ | $-CH_3$ | $-CH_3$ | H | $4-CH_3$ | 157 | 2 |
| 19 | $-C_2H_5$ | $-CH_3$ | $-CH_3$ | $-CH_3$ | $4-CH_3$ | 152 | 2 |
| 20 | $-C_2H_5$ | $-CH_3$ | $-CH_3$ | H | 4-Cl | 185-186 | 2 |
| 21 | $-CH_3$ | $-CH_3$ | $-CH_3$ | H | 2-Cl | 188 | 2 |
| 22 | $-CH_3$ | $-CH_3$ | $-CH_2-C{\equiv}CH$ | H | H | 161 | 2 |
| 23 | $-C_2H_5$ | $-CH_3$ | $-CH_3$ | H | $2-CH_3$ | 161-162 | 2 |

Patentansprüche:

1. 8-Aryl-5,6,7,8-tetrahydropyrazolo(3,4-b)(1,5)diazepin-1H,4H-5,7-dione der Formel

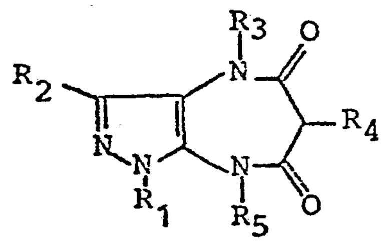

I

worin

$R_1$ und $R_2$ gleich oder verschieden sind und Wasserstoffatome, Alkylgruppen mit 1-6 C-Atomen darstellen, wobei einer der Reste $R_1$ und $R_2$ jeweils auch eine Benzyl- oder Phenylgruppe sein kann,

$R_3$ ein Wasserstoffatom, eine Alkylgruppe mit 1-6 C-Atomen, die gegebenenfalls durch eine Alkoxygruppe mit 1-6 C-Atomen oder eine Dialkylaminogruppe mit 2-12 C-Atomen oder eine Cycloalkylgruppe mit 3-6 C-Atomen substituiert ist, oder eine Alkenyl- oder Alkinylgruppe mit 2-6 C-Atomen, eine Cycloalkylgruppe mit 3-6 C-Atomen, oder eine Carbalkoxygruppe mit 2-6 C-Atomen bedeutet,

$R_4$ ein Wasserstoffatom, eine Alkylgruppe mit 1-6 C-Atomen, eine Phenylgruppe, eine Hydroxygruppe, eine Alkoxygruppe mit 1-6 C-Atomen, eine Carbalkoxygruppe mit 2-6 C-Atomen, eine Acylgruppe mit 2-6 C-Atomen, eine Aminogruppe, eine Alkylaminogruppe mit 1-7 C-Atomen oder eine Dialkylaminogruppe mit 2-12 C-Atomen oder eine Carbamoylgruppe, die eine Aminogruppe, eine Alkylaminogruppe mit 1-6 C-Atomen oder eine Dialkylaminogruppe mit 2-12 C-Atomen trägt, ist,

$R_5$ eine Phenylgruppe, eine ein- oder zweifach durch Methyl, Cl, Br, F, Nitro, Cyan und/oder Trifluormethyl substituierte Phenylgruppe oder eine 2-Pyridylgruppe sein kann.

2. Verbindungen gemäß Anspruch 1 worin

$R_1$ Wasserstoff, $CH_3$, $C_2H_5$ oder Phenyl

$R_2$ $CH_3$

$R_3$ Wasserstoff

$R_4$ Wasserstoff

$R_5$ Phenyl, 2- oder 3-Chlorphenyl

bedeuten.

3. Verbindungen gemäß Anspruch 2 worin

$R_3$ eine niedere Alkyl-, Alkenyl-, Alkinyl- oder Cycloalkylgruppe

bedeutet.

4. Verfahren zur Herstellung von Verbindungen gemäß Anspruch 1, dadurch gekennzeichnet, daß man

a) eine Verbindung der allgemeinen Formel

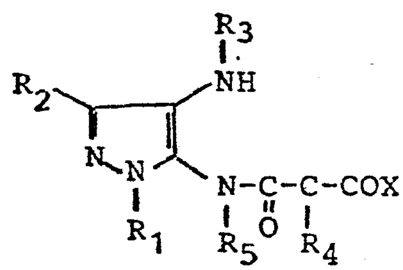

II

wobei $R_1$, $R_2$, $R_3$, $R_4$ und $R_5$ die oben angegebenen Bedeutungen haben und X eine Hydroxygruppe, eine Mercaptogruppe, ein Halogenatom, eine Alkoxygruppe, eine Alkylmercaptogruppe, eine Aminogruppe, eine Alkylaminogruppe, eine Dialkylaminogruppe, eine Benzoyloxygruppe, eine Aryloxygruppe, eine Acyloxygruppe oder die Gruppe $N_3$ bedeutet, zum Siebenring zyklisiert; oder

b) eine Verbindung der allgemeinen Formel

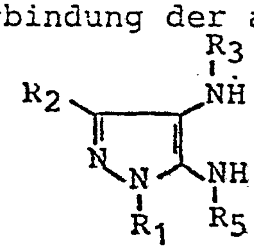

III

BAD ORIGINAL

wobei $R_1$, $R_2$, $R_3$ und $R_5$ die oben angegebene Bedeutung haben, mit einem aktivierten Malonsäurederivat, z.B. Mabnsäuredihalogenid, Malonester, Kohlensuboxyd, Meldrumsäure oder mit Malonsäure selbst zum Siebenring kondensiert; oder

c) eine Verbindung der allgemeinen Formel

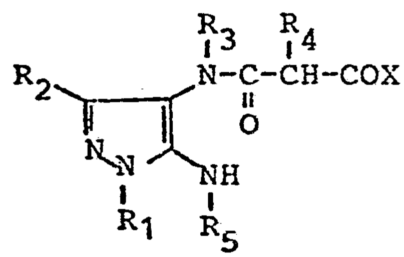

IV

wobei $R_1$, $R_2$, $R_3$, $R_4$ und $R_5$ die oben angegebene Bedeutung haben und für X die unter a) angegebenen Gruppen in Frage kommen, mit den unter a) beschriebenen Verfahren zum Siebenring zyklisiert, und gegebenenfalls in den erhaltenen Verbindungen, in denen einer oder beide der Reste $R_3$ und $R_4$ Wasserstoff sind, in an sich bekannter Weise diese Positionen gleichzeitig oder nacheinander durch Alkylierung oder Acylierung substituiert.

6. Verfahren zur Herstellung von Arzneimitteln mit anxiolytischer und antikonvulsiver Wirkung, dadurch gekennzeichnet, daß man Verbindungen gemäß Anspruch 1 gegebenenfalls zusammen mit pharmazeutischen Trägern oder Hilfsstoffen in eine für therapeutische Zwecke geeignete Anwendungsform bringt.

7. Pharmazeutisches Präparat mit anxiolytischer und antikonvulsiver Wirkung, enthaltend als Wirkstoff eine oder mehrere Verbindungen der allgemeinen Formel I in Mengen von 1 - 50 mg, vorzugsweise 5 - 25 mg/Dosis im Gemisch mit üblichen pharmazeutischen Hilfs- und Trägerstoffen.

Patentansprüche für Österreich:

1. Verfahren zur Herstellung von 8-Aryl-5,6,7,8-tetrahydropyrazolo(3,4-b)(1,5)diazepin-1H,4H-5,7-dione der Formel

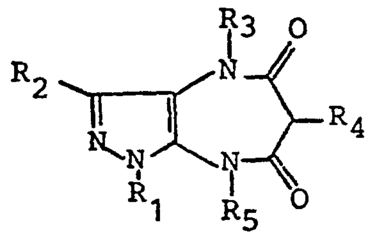

I

worin

$R_1$ und $R_2$ gleich oder verschieden sind und Wasserstoffatome, Alkylgruppen mit 1-6 C-Atomen darstellen, wobei einer der Reste $R_1$ und $R_2$ jeweils auch eine Benzyl- oder Phenylgruppe sein kann,

$R_3$ ein Wasserstoffatom, eine Alkylgruppe mit 1-6 C-Atomen, die gegebenenfalls durch eine Alkoxygruppe mit 1-6 C-Atomen oder eine Dialkylaminogruppe mit 2-12 C-Atomen oder eine Cycloalkylgruppe mit 3-6 C-Atomen substituiert ist, oder eine Alkenyl- oder Alkinylgruppe mit 2-6 C-Atomen, eine Cycloalkylgruppe mit 3-6 C-Atomen, oder eine Carbalkoxygruppe mit 2-6 C-Atomen bedeutet,

$R_4$ ein Wasserstoffatom, eine Alkylgruppe mit 1-6 C-Atomen, eine Phenylgruppe, eine Hydroxygruppe, eine Alkoxygruppe mit 1-6 C-Atomen, eine Carbalkoxygruppe mit 2-6 C-Atomen, eine Acylgruppe mit 2-6 C-Atomen, eine Aminogruppe, eine Alkylaminogruppe mit 1-7 C-Atomen oder eine Dialkylaminogruppe mit 2-12 C-Atomen oder eine Carbamoylgruppe, die eine Aminogruppe, eine Alkylaminogruppe mit 1-6 C-Atomen oder eine Dialkylaminogruppe mit 2-12 C-Atomen trägt, ist,

$R_5$ eine Phenylgruppe, eine ein- oder zweifach durch Methyl, Cl, Br, F, Nitro, Cyan und/oder Trifluormethyl substituierte Phenylgruppe oder eine 2-Pyridylgruppe sein kann,

dadurch gekennzeichnet, dass man

a) eine Verbindung der allgemeinen Formel

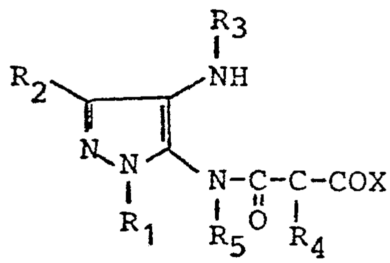

II

wobei $R_1$, $R_2$, $R_3$, $R_4$ und $R_5$ die oben angegebenen Bedeutungen haben und X eine Hydroxygruppe, eine Mercaptogruppe, ein Halogenatom, eine Alkoxygruppe, eine Alkylmercaptogruppe, eine Aminogruppe, eine Alkylaminogruppe, eine Dialkylaminogruppe, eine Benzoyloxygruppe, eine Aryloxygruppe, eine Acyloxygruppe oder die Gruppe $N_3$ bedeutet, zum Siebenring zyklisiert; oder

b) eine Verbindung der allgemeinen Formel

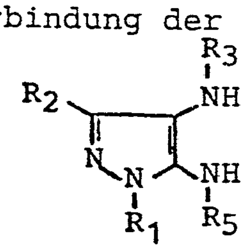

III

wobei $R_1$, $R_2$, $R_3$ und $R_5$ die oben angegebene Bedeutung haben, mit einem aktivierten Malonsäurederivat, z.B. Malonsäuredihalogenid, Malonester, Kohlensuboxyd, Meldrumsäure oder mit Malonsäure selbst zum Siebenring kondensiert; oder

c) eine Verbindung der allgemeinen Formel

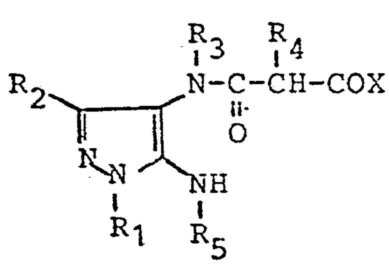

IV

wobei $R_1$, $R_2$, $R_3$, $R_4$ und $R_5$ die oben angegebene Bedeutung haben und für X die unter a) angegebenen Gruppen in Frage kommen, mit den unter a) beschriebenen Verfahren zum Siebenring zyklisiert, und gegebenenfalls in den erhaltenen Verbindungen, in denen einer oder beide der Reste $R_3$ und $R_4$ Wasserstoff sind, in an sich bekannter Weise diese Positionen gleichzeitig oder nacheinander durch Alkylierung oder Acylierung substituiert.

2. Verfahren zur Herstellung von Arzneimitteln mit anxiolytischer und antikonvulsiver Wirkung, dadurch gekennzeichnet, dass man Verbindungen gemäss Anspruch 1 gegebenenfalls zusammen mit pharmazeutischen Trägern oder Hilfsstoffen in eine für therapeutische Zwecke geeignete Anwendungsform bringt.

BAD ORIGINAL

0014959

Europäisches Patentamt

# EUROPÄISCHER RECHERCHENBERICHT

Nummer der Anmeldung
EP 80 10 0738

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | DE - A - 2 360 852 (DEUTSCHE GOLD- UND SILBER-SCHEIDEANSTALT)<br>* Ansprüche 4,9 *<br>---- | 1,7 | C 07 D 487/04<br>A 61 K 31/55<br>C 07 D 231/38<br>231/40<br>(C 07 D 487/04<br>243/00<br>231/00) |
| | | | RECHERCHIERTE SACHGEBIETE (Int. Cl. 3) |
| | | | C 07 D 487/04<br>A 61 K 31/55 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| | Der vorliegende Recherchenbericht wurde für alle Patentansprüche erstellt. | | |

| Recherchenort | Abschlußdatum der Recherche | Prüfer |
|---|---|---|
| Den Haag | 09-05-1980 | ALFARO |

EPA form 1503.1 06.78